(19)

Europäisches Patentamt
European Patent Office
Office européen des brevets

(11) **EP 3 933 672 A1**

(12) **EUROPEAN PATENT APPLICATION**

(43) Date of publication:
**05.01.2022 Bulletin 2022/01**

(51) Int Cl.:
***G06K 9/00*** *(2006.01)*          ***G06K 9/03*** *(2006.01)*

(21) Application number: **21155695.6**

(22) Date of filing: **08.02.2021**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **01.07.2020  CN 202010620041**

(71) Applicant: **Wuhan Endoangel Medical Technology Co., Ltd.**
**Wuhan, Hubei 430000 (CN)**

(72) Inventors:
- **LI, Chao**
  **Wuhan, Hubei 430000 (CN)**
- **LIU, Jinlong**
  **Wuhan, Hubei 430000 (CN)**
- **LIU, Qiwei**
  **Wuhan, Hubei 430000 (CN)**
- **HU, Shan**
  **Wuhan, Hubei 430000 (CN)**

(74) Representative: **Niburska, Danuta**
**Kancelaria Patentowa**
**Al. 3 Maja 68 B**
**76-200 Slupsk (PL)**

(54) **METHOD OF AUTOMATIC IMAGE FREEZING OF DIGESTIVE ENDOSCOPY**

(57)     A method of automatic image freezing of digestive endoscopy based on a perceptual hash algorithm includes: analyzing a video streaming of digestive endoscopy acquired by digestive endoscopy imaging system into image data; calculating a similarity between an image at t point in time and images of first n frames, to obtain a weighted similarity k of the image; and comparing the weighted similarity *k* of the image at *t* point in time with a freezing boundary l, and triggering an instruction of image freezing when the k reaches / to obtain the clear images with the best visual field from the video streaming of digestive endoscopy.

FIG. 1

**Description**

This disclosure relates to the field of medical image processing technology, and more particularly to a method of automatic image freezing of digestive endoscopy based on a perceptual hash algorithm.

Digestive endoscope is a set of equipment for the diagnosis and treatment of digestive diseases by means of images obtained from the digestive tract or by ultrasound and X-ray images of the digestive tract and digestive organs. In the process of digestive endoscopy, the digestive endoscopy imaging system (combined with digestive endoscopy, display system and computer workstation) inputs the digestive endoscopy image into the computer for digital processing, and displays the output image in real time, which can be used for image freezing, acquisition and storage. Among them, image freezing is a common basic function commonly used in the process of digestive endoscopy. Endoscopists freeze real-time images by pressing the "freeze" button of digestive endoscope equipment to obtain static images for careful observation.

For frozen images, the clearest and the most stable frame should be captured in the endoscopic videos at the current moment. At present, the frozen images may be fuzzy. To ensure the clarity of frozen images, relevant personnel put forward relevant views. In the related art titled "a method, device, equipment of image freezing and computer-readable storage medium" of Chinese Patent Publication Number CN108063920A proposes a method of image freezing, in which the image with the highest definition value is fused with the previous frame image and the latter frame image to generate the final frozen image, so as to make the frozen image clearer and avoid the loss of useful image information. This method must rely on the endoscopists to press the button to issue the freezing instruction. Due to the limited factors such as the low operational level and slow reaction speed of the endoscopists, the visual field in the videos during freezing is deviated, and the frozen images is not the effective area images that the endoscopists want.

The disclosure provides a method of automatic image freezing of digestive endoscopy based on a perceptual hash algorithm.

Specifically, the method of automatic image freezing of digestive endoscopy based on a perceptual hash algorithm comprises:

1) analyzing a video streaming of digestive endoscopy acquired by digestive endoscopy imaging system into image data;

2) calculating a similarity between an image at $t$ point in time and images of first $n$ frames, to obtain a weighted similarity $k$ of the image;

3) comparing the weighted similarity $k$ of the image at $t$ point in time with a freezing boundary $l$, and triggering an instruction of image freezing when the k reaches / to obtain static images from the video streaming of digestive endoscopy.

In a class of this embodiment, in 1), the method further comprises removing fuzzy invalid frame images, cropping clear images, reducing a size of cropped images, retaining the image structure information, and converting the cropped images into gray scale images.

In a class of this embodiment, in 1), bicubic interpolation is adopted to reduce the size of the cropped images.

In a class of this embodiment, in 1), a calculation formula of converting the cropped images into the gray scale images is as follows:

$$Gray = 0.30*R + 0.59*G + 0.11*B;$$

where R, G and B respectively represent information values of red light, green light and blue light.

In a class of this embodiment, in 1), the Gray-scale value of adjacent pixels in each line of the gray image are compared. If the gray value of the previous pixel is greater than that of the latter pixel, the dHash value is set to "1", if not, the dHash value is set to "0".

In a class of this embodiment, in 2), the similarity between different images is calculated by calculating a Hamming distance between different images.

In a class of this embodiment, in 2), the Hamming distance between different images refers to a number of digits required to change dHash values corresponding to a first image to dHash values corresponding to a second image. For example, the Hamming distance between 0110 and 1111 is 2.

In a class of this embodiment, in 2), a formula for calculating the similarity between a current image and the first $n$ frames is as follows:

$$Sim = 100 * (64 - d (x, y)) / 64;$$

where the d (x, y) is the Hamming distance between different images, d (x, y)=$\Sigma$ x $\oplus$y, x and y are the dHash values corresponding to different images, and $\oplus$ is exclusive OR.

In a class of this embodiment, in 3), the freezing boundary *l* is obtained by analyzing the video of manually freezing image by endoscopist during the digestive endoscopy.

In a class of this embodiment, in 3), when $k \geq l$, it is judged that the image is frozen at *t* point in time, then the image freezing command is triggered at *t time* point, that is, the image can be frozen without endoscopist's operation of "freezing"; when $k < l$, it is judged that *t* point time is not a frozen image, then the command of freezing image at t point time is not triggered.

The following advantages are associated with the method of automatic image freezing of digestive endoscopy of the disclosure. Using this method, when endoscopists need to carefully examine the image of a certain visual field, they only need to stop the movement of the endoscopic body to keep the visual field unchanged. Then the images can be automatically determined as frozen images. There is no need for the endoscopists to manually operate the "freeze" button, thus reducing the workload of the endoscopists. The system automatically executes the freezing instruction, which can avoid the deviation of visual field or loss of effective information of frozen images due to slow reaction or unskilled operation, thus effectively acquiring the clear images with the best visual field.

FIG. 1 is a flow chart of a method of automatic image freezing of digestive endoscopy according to one embodiment of the disclosure;

FIG. 2 is a schematic diagram of scaling images by bicubic interpolation according to one embodiment of the disclosure;

FIG. 3 is s schematic diagram of a pixel (x, y) in a target interpolation graph which is the closest mapping point in the original images according to one embodiment of the disclosure; and

FIG. 4 is a gray scale image according to one embodiment of the disclosure.

To further illustrate the disclosure, embodiments detailing a method of automatic image freezing of digestive endoscopy based on a perceptual hash algorithm are described below. It should be noted that the following embodiments are intended to describe and not to limit the disclosure.

Image structure information: refers to the hue change and position arrangement of each pixel in the image.

Gray-scale value: the black tone is used to represent the color of image, gray-scale is the brightness of pixel is divided into 256 grades from 0 to 255. Gray-scale value is the number from 0 to 255, 0 represents black, and 255 represents white.

Gray scale image: the image is composed of every pixel represented by Gray-scale value.

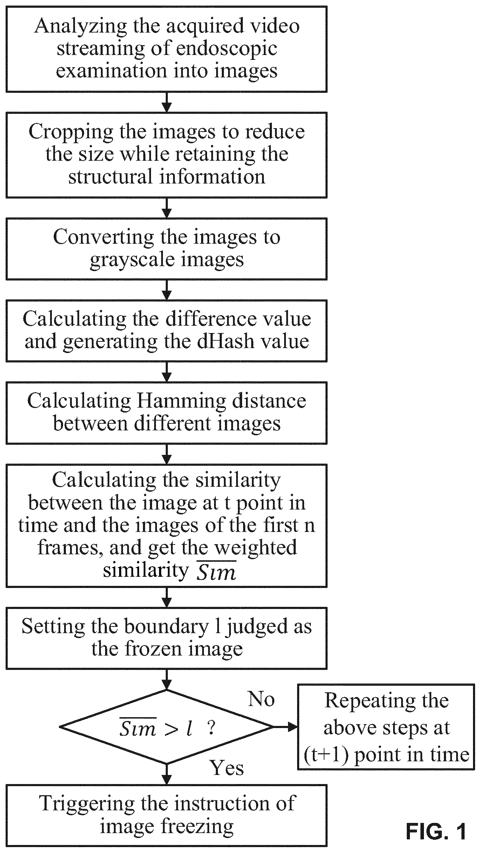

As shown in FIGS. 1-4, the disclosure provides a method of automatic image freezing of digestive endoscopy based on a perceptual hash algorithm, the method comprising:

S1. analyzing a video streaming of digestive endoscopy acquired by digestive endoscopy imaging system into image data;

S2. calculating a similarity between an image at *t* point in time and images of first *n* frames, to obtain a weighted similarity *k* of the image; and

S3. comparing the weighted similarity *k* of the image at *t* point in time with a freezing boundary *l*, and triggering an instruction of image freezing when the *k* reaches *l* to obtain the clear images with the best visual field from the video streaming of digestive endoscopy.

Example 1

S1. Obtaining the video streaming of digestive endoscopy through the digestive endoscopy imaging system, and analyzing the video streaming into images (30 frames per second). Then remove fuzzy invalid frame images and take 10 of them;

S2. Cropping the valid frame images to 360*360 pixels, further reducing the size of cropped images, and only retaining the structural information of images;

An image with 360*360 pixel has more than 100,000 pixels, containing a huge amount of information, and many details need to be processed. Therefore, the image is required to be scaled to a very small size. The purpose is to remove the details of the image, and only retain the basic information such as structure, light and shade, and discard the differences caused by different sizes and proportions.

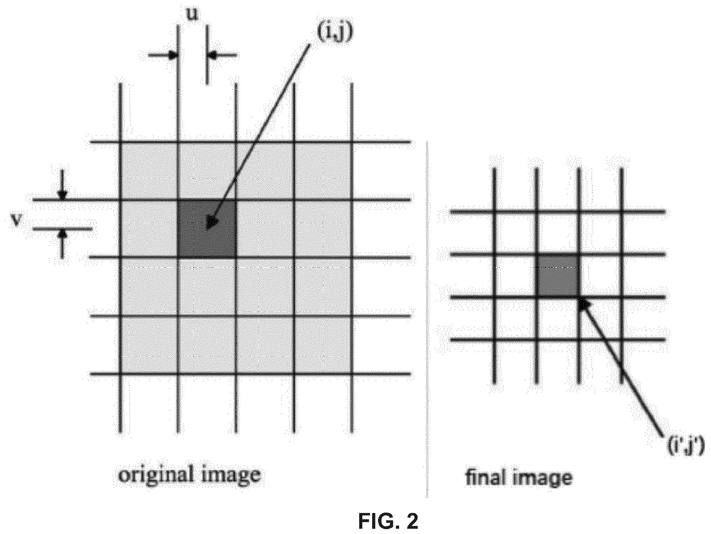

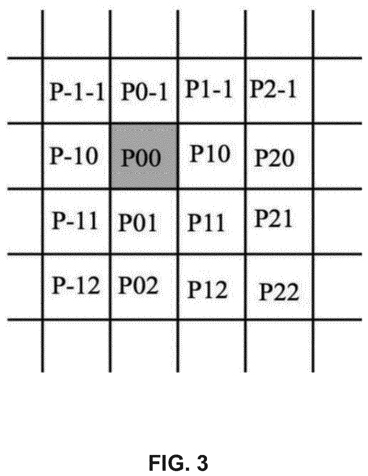

The bicubic interpolation is adopted to scale the image. Although the calculation is large, the quality of the scaled image is high and the image is not easy to be distorted. According to FIG. 2 and the mathematical expression of the bicubic interpolation, it can be seen that the pixel value corresponding to the coordinate point ($i'$, $j'$) in the reduced image after the interpolation is the sum of the weight convolution of the adjacent 16 pixel points at ($i$, $j$) in the original image. P00 in FIG. 3 represents a pixel $(x, y)$ in the target interpolation graph which is the closest mapping point in the original images. If the expression of pixel value of each coordinate point of ($i$, $j$) in the original image is $f$ ($i$, $j$), then the pixel value of corresponding coordinate after interpolation is F ($i'$, $j'$), it can be obtained by the following formula:

$$F(i',j') = \sum_{row=-1}^{2} \sum_{col=-1}^{2} f(i+row, j+col) S(row-v) S(col-u)$$

where $v$ represents the deviation of the number of rows, $u$ represents the deviation of the number of columns; $row$ represents a row, $col$ represents a column; $S(x)$ represents the interpolation expression, comprising common expressions based on trigonometric values, Bell distribution, and B-spline curve, which can be selected according to different needs. The Bell distribution expression is selected in the embodiment of the disclosure.

In order to better calculate the dHash value of the converted images, the embodiment of the disclosure reduces the images to 9*8, a total of 72 pixels.

Converting the images to gray scale images;

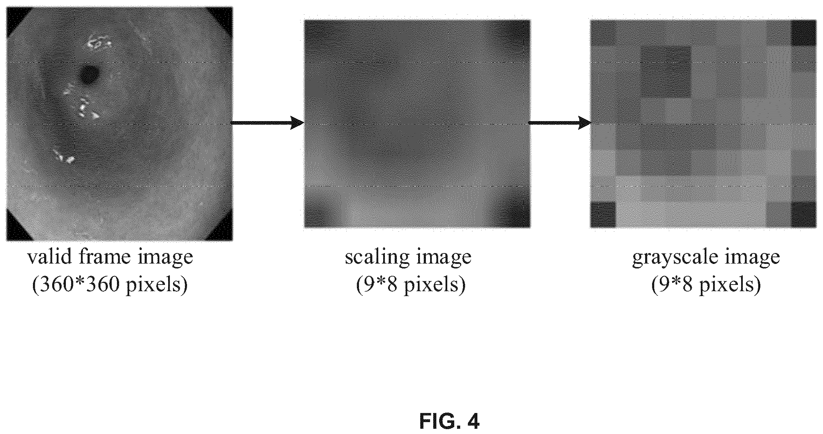

The reduced images are color and consists of RGB values represented as $(R, G, B)$. R, G and B are the information values of red light, green light and blue light respectively. The larger the value is, the brighter the color is, while the smaller the value is, the darker the color is. For example, white represents $(255,255,255)$ and black represents $(0,0,0)$. In general, there is little relationship between image similarity and color. Therefore, the image is processed into gray scale image to reduce the complexity of later calculation, referring to the final obtained gray scale image with 9*8 pixel in FIG. 4. RGB values can be converted to Gray-scale value (represented only by an integer between 0 and 255).

The weighted average method is adopted: due to the different sensitivity of human eyes to red, green and blue, different weights are given to each pixel of the images to calculate the grays values. The formula is as follows:

$$Gray = 0.30 * R + 0.59 * G + 0.11 * B$$

Comparing the gray difference of pixels of gray scale images, calculate the difference values, and generate the dHash values of images.

The gray scale images have 9 pixels per row for a total of 8 rows. Comparing the difference between two adjacent pixels in each row, and each row generates eight difference values. If the gray value of the previous pixel is greater than that of the latter pixel, the difference value is set to "1", if not, the difference value is set to "0". Then the calculated difference values of the pixels are compared from top to bottom and from left to right, and splice them into 64-bit binary string in order, which is the dHash values of the images.

Example 2

The example is basically the same as that in Example 1 except the following descriptions.

In S2, the similarity between different images is calculated by calculating the Hamming distance between different images. The Hamming distance between different images represents the number of digits required to change dHash values corresponding to image A to dHash values corresponding to image B. The formula to calculate the similarity between the current image and the first $n$ frames is:

$$Sim = 100 * (64 - d (x, y)) / 64;$$

where the $d (x, y)$ is the Hamming distance between different images, $d (x, y) = \sum x \oplus y$, $x$ and $y$ are the dHash values corresponding to different images, and ⊕ is exclusive OR.

Calculating the Hamming distance between different images;

Hamming distance represents the number of different characters in the corresponding position of two equal length strings, which in dHash is to take the binary dHash value of two images to exclusive OR and calculate the digit of "1" of the exclusive OR result, that is, the digit with different binary dHash values. The Hamming distance between the strings x and y is defined as *d (x, y):*

$$d(x, y) = \sum x \oplus y$$

⊕ is exclusive OR; *x* and *y* are the dHash values corresponding to different images.

S6. Comparing the dHash values of the image at t point in time and the images of the first 9 frames to obtain the overlap rate of the current image and the images of the first 9 frames respectively, namely, the similarity. The calculation formula of similarity *Sim* of two images is *Sim= 100 \* (64 - d (x, y)) / 64*. And the weighted similarity of image at *t* point in time is obtained, $\overline{Sim} = \sum_{i=1}^{9} \frac{i}{45} * Sim_i$, $Sim_i$ represents the similarity between the image *t* point in time and the images of the first *i* frames (*i* value range is 1-9).

Example 3

The example is basically the same as that in Example 2 except the following descriptions.

The freezing boundary / of the weighted similarity is set by analyzing the video of manually freezing image by endoscopist during digestive endoscopy.

The weighted similarity $\overline{Sim}$ of the image at *t* point in time was compared with the cutoff *l*. When $\overline{Sim} \leq l$, the image is judged as frozen image at *t* point in time and triggering an instruction of image freezing at t point in time; When $\overline{Sim} > l$, the image can't be judged as frozen image at *t* point in time and can't trigger the instruction of image freezing at t point in time, and repeat the above steps at the next *(t + 1)* point in time.

This technical scheme is used to replace the operation of manually freezing image, which can not only effectively obtain the clear image of the best visual field, but also reduce the workload of endoscopist. The core is how to trigger the instruction of image freezing. Based on the habit of human operation, when endoscopist want to capture static images for freezing operation, they will try their best to keep the endoscopic body and the examination area to remain relatively static. The similarity of sequent frames in the output videos is very high. The perceptual hash algorithm (hereinafter referred to as PHA) is a kind of hash algorithm, which is mainly used to search similar images. PHA is a general name of a class of hash algorithm, whose function is to generate the "fingerprint" string of each image and compare the fingerprint information of different images to judge the similarity of images. The closer the results are, the more similar the images are. PHA comprises average hash (aHash), perceptual hash (pHash) and different hash (dHash).

From the above analysis, PHA is used to analyze and calculate the similarity of the adjacent frames in the per unit time of the digestive endoscopic images. The higher the similarity is, the more likely the image will be frozen. When the similarity reaches the preset boundary, it can be considered as freezing operation, which can automatically issue the freezing instruction, and complete the subsequent process.

The disclosure enables endoscopists only need to stop the movement of endoscopic body to keep the visual field unchanged when they need to carefully examine the image of a certain visual field. Then the images can be automatically determined as frozen images. There is no need for the endoscopists to manually operate the "freeze" button, so as to reduce the workload of the endoscopists. The system automatically executes the freezing instruction, which can avoid the deviation of visual field or loss of effective information of frozen images due to slow reaction or unskilled operation, so as to effectively obtain the clear images with the best visual field.

**Claims**

1. A method of automatic image freezing of digestive endoscopy, the method comprising:

   1) analyzing a video streaming of digestive endoscopy acquired by a digestive endoscopy imaging system into image data;
   2) calculating a similarity between an image at *t* point in time and images of first *n* frames, to obtain a weighted similarity *k* of the image; and

3) comparing the weighted similarity *k* of the image at *t* point in time with a freezing boundary *l*, and triggering an instruction of image freezing when the *k* reaches *l* to obtain clear images with a best visual field from the video streaming of digestive endoscopy.

2. The method of claim 1, wherein in 1), the method further comprises removing fuzzy invalid frame images, cropping the clear images, reducing a size of cropped images, retaining image structure information, and converting the cropped images into gray scale images.

3. The method of claim 2, wherein in 1), bicubic interpolation is adopted to reduce the size of the cropped images.

4. The method of claim 2, wherein in 1), a calculation formula of converting the cropped images into the gray scale images is as follows:

$$Gray = 0.30*R + 0.59*G + 0.11*B;$$

where R, G and B respectively represent information values of red light, green light and blue light.

5. The method of claim 2, wherein in 1), Gray-scale value of adjacent pixels in each line of a gray image are compared; if a Gray-scale value of a previous pixel is greater than that of a latter pixel, a dHash value is set to "1", if not, the dHash value is set to "0".

6. The method of claim 1, wherein in 2), the similarity between different images is calculated by calculating a Hamming distance between different images.

7. The method of claim 6, wherein in 2), the Hamming distance between different images refers to a number of digits required to change dHash values corresponding to a first image to dHash values corresponding to a second image.

8. The method of claim 7, wherein in 2), a formula for calculating the similarity between a current image and the first *n* frames is as follows:

$$Sim = 100 * (64 - d(x, y)) / 64;$$

where d (x, y) is the Hamming distance between different images, d (x, y)=$\Sigma$ x $\oplus$y, x and y are the dHash values corresponding to different images, and $\oplus$ is exclusive OR.

9. The method of claim 1, wherein in 3), the freezing boundary *l* is obtained by analyzing a video of manually freezing image by an endoscopist during the digestive endoscopy.

Analyzing the acquired video streaming of endoscopic examination into images

↓

Cropping the images to reduce the size while retaining the structural information

↓

Converting the images to grayscale images

↓

Calculating the difference value and generating the dHash value

↓

Calculating Hamming distance between different images

↓

Calculating the similarity between the image at t point in time and the images of the first n frames, and get the weighted similarity $\overline{Sim}$

↓

Setting the boundary l judged as the frozen image

↓

$\overline{Sim} > l$ ? — No → Repeating the above steps at (t+1) point in time

Yes

↓

Triggering the instruction of image freezing

**FIG. 1**

FIG. 2

| | | | |
|---|---|---|---|
| P-1-1 | P0-1 | P1-1 | P2-1 |
| P-10 | P00 | P10 | P20 |
| P-11 | P01 | P11 | P21 |
| P-12 | P02 | P12 | P22 |

FIG. 3

valid frame image
(360*360 pixels)

scaling image
(9*8 pixels)

grayscale image
(9*8 pixels)

**FIG. 4**

Europäisches
Patentamt
European
Patent Office
Office européen
des brevets

# EUROPEAN SEARCH REPORT

**Application Number**

EP 21 15 5695

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2014 220690 A (OLYMPUS MEDICAL SYSTEMS CORP) 20 November 2014 (2014-11-20) | 1,9 | INV. G06K9/00 G06K9/03 |
| Y | * paragraphs [0032;0077] - [0094] * | 2-8 | |
| X | US 2017/353666 A1 (NUMATA KENJI [JP]) 7 December 2017 (2017-12-07) * paragraphs [0003] - [0007;0049]; figure 6 * | 1 | |
| Y | WO 2020/114037 A1 (WUHAN ENDOANGEL MEDICAL TECH CO LTD [CN]) 11 June 2020 (2020-06-11) * paragraphs [0005] - [0069] * | 2-8 | |

TECHNICAL FIELDS
SEARCHED      (IPC)

G06K
A61B

The present search report has been drawn up for all claims

| Place of search | Date of completion of the search | Examiner |
|---|---|---|
| The Hague | 24 June 2021 | Craciun, Paula |

EPO FORM 1503 03.82 (P04C01)

## ANNEX TO THE EUROPEAN SEARCH REPORT
## ON EUROPEAN PATENT APPLICATION NO.

EP 21 15 5695

This annex lists the patent family members relating to the patent documents cited in the above-mentioned European search report.
The members are as contained in the European Patent Office EDP file on
The European Patent Office is in no way liable for these particulars which are merely given for the purpose of information.

24-06-2021

| Patent document cited in search report | | Publication date | Patent family member(s) | | Publication date |
|---|---|---|---|---|---|
| JP 2014220690 | A | 20-11-2014 | NONE | | |
| US 2017353666 | A1 | 07-12-2017 | JP | 6767383 B2 | 14-10-2020 |
| | | | JP | WO2017086091 A1 | 30-08-2018 |
| | | | US | 2017353666 A1 | 07-12-2017 |
| | | | WO | 2017086091 A1 | 26-05-2017 |
| WO 2020114037 | A1 | 11-06-2020 | CN | 109598716 A | 09-04-2019 |
| | | | EP | 3848891 A1 | 14-07-2021 |
| | | | US | 2020364880 A1 | 19-11-2020 |
| | | | WO | 2020114037 A1 | 11-06-2020 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 108063920 A